# FASCICULE DE BREVET EUROPEEN

(11) **EP 3 282 850 B1**
(45) Date de publication et mention de la délivrance du brevet: **15.01.2020**
(21) Numéro de dépôt: 16719305.1
(22) Date de dépôt: 15.04.2016
(51) Int. Cl.: A01N 65/10, A01P 5/00

(54) **RÉSISTANCE À HETERODERA CAROTAE ET MÉTHODES D'UTILISATION**
RESISTENZ GEGEN HETERODERA CAROTAE UND VERWENDUNGSVERFAHREN
RESISTENCE AGAINST HETERODERA CAROTAE AND METHODS OF USE

(30) Priorité: 17.04.2015 FR 1553449
(43) Date de publication de la demande: 21.02.2018
(73) Titulaire: Vilmorin & Cie, 75001 Paris (FR)
(72) Inventeur: BARROT, Laure, 49250 La Menitre (FR)
(74) Mandataire: Sellin, Carole
(86) Numéro de dépôt international: PCT/EP2016/058304
(87) Numéro de publication internationale: WO 2016/166262

(56) Documents cités:
- WO-A1-2005/063989
- US-A- 4 442 092

## Description

La présente description est relative à une plante de la famille *Daucus carota* présentant une activité nématicide contre le nématode à kyste *Heterodera carotae,* de préférence n'appartenant pas à la sous-espèce des carottes cultivées *Daucus carota* subsp. *sativus.* La présente invention est aussi relative aux utilisations d'une telle plante *Daucus carota* nématicide, notamment pour diminuer le niveau d'infestation d'un champ de culture infesté par le nématode à kyste *Heterodera carotae.*

Les carottes appartiennent à la grande famille des apiacées, anciennement connues sous le nom d'ombellifères. Cette plante herbacée bisannuelle est cultivée pour sa racine charnue et comestible qui est elle-même appelée carotte.
Selon les statistiques de l'organisation mondiale de l'alimentation (FAO), la production mondiale de carotte était en 2007 de plus de 26 millions de tonnes, la moitié provenant de trois pays, la Chine, les Etats-Unis et la Russie.
La production Française est d'environ 600 000 tonnes, dont une grande partie, 350.000 tonnes, est destinée au marché de frais (primeur et conservation), le reste étant dévolu à la production des carottes destinées à l'industrie.
Si la production des carottes concerne l'ensemble du territoire français, elle est surtout concentrée sur quatre grandes zones, l'Ouest de la France (Normandie et Bretagne), l'Aquitaine (les Landes), le bassin Méditerranéen et la région Nord Picardie. Les deux premières zones sont particulièrement favorables notamment à cause des terrains sablonneux qui sont propices au développement harmonieux des racines et à la mécanisation de la récolte.
A elle seule, la production française destinée au marché de frais représente une surface de plus de 8500 hectares.
La culture de la carotte est une culture de plein champ qui fait face à de nombreuses agressions. La protection phytosanitaire de la culture de la carotte reste le centre d'intérêt majeur des producteurs. Les principales difficultés viennent, pour les maladies et les ravageurs telluriques des nématodes, des pythiums, de *Rhizoctonia solani* et de la mouche de la carotte (*Psila rosae*)*,* et, pour les maladies et ravageurs aériens du puceron *cavalriella aegopodii* et d'*Alternaria dauci.*
Le nématode à kyste, *Heterodera carotae* est responsable de pertes de rendement important liées soit au ralentissement de la production, soit à une réduction de la taille de la racine combinée à une forte production des radicelles latérales, donnant un aspect chevelu assez caractéristique mais dépréciant fortement le produit qui ne peut plus être commercialisé. Dans les zones de production en terrain sablonneux, les pertes liées à *Heterodera carotae* peuvent avoisiner les 90%.

Les méthodes de luttes chimiques sont aujourd'hui les méthodes les plus efficaces pour désinfecter les champs. Ainsi, le 1,3 dichloropropène entraîne des réductions de population de nématodes sur les sols traités de 80% en moyenne. Toutefois, dans un contexte où les conditions de cultures deviennent de plus en plus respectueuses de l'environnement, ces traitements chimiques fumigants et/ou les nématicides dans le cas des nématodes, posent des problèmes environnementaux et leur emploi devrait décroître : le 1,3 dichloropropène vient d'être interdit au niveau Européen, laissant les agriculteurs sans solutions de lutte chimique efficace pour les prochains semis.
De plus, on constate que si ces traitements chimiques permettent la culture de la carotte en terrain infesté, ils ne provoquent pas pour autant une diminution stable des populations de nématodes. En effet, les individus, principalement sous forme de kyste, restant après un traitement suffisent à créer une nouvelle population infestante dès la mise en place d'une nouvelle culture. Il est donc nécessaire de renouveler continuellement ces traitements chimiques, ce qui s'avère onéreux et pose là encore des problèmes environnementaux.
Divers autres moyens de protection sont disponibles, par exemple la désinfection des sols par la vapeur ou par solarisation. Avec des réductions constatées de 50% des populations de nématodes, ces méthodes ne sont pas optimales et s'avèrent souvent difficiles d'emploi.
La rotation des cultures est une possibilité qui peut être envisagée pour combattre certains pathogènes, mais elle est peu adaptée dans le cas des nématodes à kystes qui ont la particularité, en l'absence de carottes, de se conserver de nombreuses années dans le sol à l'état de vie ralentie, sous la forme de kyste qui écloront dès la mise en place d'une nouvelle culture de carotte, suite à l'émission d'exsudats racinaires. On considère qu'il faut au moins huit années sans culture de carotte sur une parcelle infestée avant d'atteindre un seuil acceptable pour une nouvelle culture, qui va elle-même relancer l'infestation pour les années suivantes.
Les méthodes culturales telles que la technique de la carotte piège permettent de réduire les populations de nématodes dans les champs infectés. Les exsudats racinaires des carottes implantées dans les champs dont on souhaite diminuer les populations de nématodes vont permettre l'éclosion des larves. Ces dernières vont migrer dans les racines, et la destruction mécanique de la culture, avant la fin d'un cycle de culture complet, va permettre d'arrêter le développement des larves. Cette technique culturale présente toutefois des limites car l'ensemble des kystes infectant un champ n'éclosent pas en même temps. Si la culture est détruite trop tôt, des kystes en nombres suffisants restent dans le champ et occasionnent des pertes de la production suivante. Si la culture est détruite trop tard, de nouvelles larves, voire de nouveaux kystes sont produits, qui occasionnent de même des pertes de la production qui suit l'utilisation du piège. Il est donc extrêmement difficile de déterminer le moment optimal auquel la culture doit être détruite et même lorsque la destruction est opérée à ce moment-là, du fait que les kystes n'ont pas tous éclos simultanément, il n'est guère possible d'obtenir un niveau de désinfestation semblable à celui obtenu avec des produits chimiques.
Des variétés de carottes résistantes au nématode à kyste, *Heterodera carotae* pourraient être une solution intéressante, toutefois, à ce jour et malgré des efforts de recherche conséquents dans ce domaine, de telles variétés n'existent pas.
Le document US 4,442,092 décrit une alternative aux produits chimiques, à savoir l'utilisation d'un extrait de plante de sésame ou de graines de sésame. Aucun effet sur le nématode *Heterodera carotae* ou sur la culture de carottes n'est cependant démontré.
Le document WO2005/063989 décrit des plantes transgéniques transformées pour exprimer par exemple une toxine nématicide sous le contrôle d'un promoteur inductible lors d'une infection par nématodes. L'application à la carotte n'est pas divulguée ni la désinfection d'un milieu de culture. Ainsi, il n'existe pas de méthode non chimique efficace de lutte contre le nématode à kyste, *Heterodera carotae.* Les présents inventeurs ont développé avec succès une technique originale utilisant des plantes *Daucus carota* nématicides permettant de diminuer suffisamment le niveau d'infestation d'un champ de culture par le nématode à kyste *Heterodera carotae* afin de permettre une culture de carotte.

La classe de nématodes comprend plus de 15000 espèces que l'on retrouve dans le sol, les eaux et qui pour certains d'entre eux ont adopté un mode de vie particulier, le parasitisme, que ce soit au dépend d'animaux ou de végétaux.
L'espèce *Heterodea carotae* a été identifiée par Jones en 1950, en Angleterre et a depuis été retrouvée dans la plupart des pays Européens où elle provoque des pertes de production importantes. C'est un endoparasite sédentaire dont le développement se déroule à l'intérieur des tissus végétaux de la carotte. Les symptômes aux champs se traduisent par des zones à végétation irrégulières, le feuillage perdant de sa vitalité, sa couleur passant au jaune-rouge avec des nécroses. Les plantes sont peu vigoureuses. La taille de la racine est réduite, concomitamment à une intense production de radicelles qui donnent un aspect chevelu à la racine. La lignification se fait plus rapidement, et la croissance est irrégulière, ce qui occasionne des distorsions et de la déformation du pivot. L'apex quant à lui se nécrose. L'attaque du nématode provoque un fort stress hydrique de la plante, les pertes de rendement pouvant éventuellement être limitées par l'irrigation de la culture ainsi que par l'apport d'engrais.
Le cycle biologique du nématode à kyste *Heterodera carotae* est caractérisé par quatre phases, commençant par l'éclosion des kystes en présence d'exsudats racinaires émis par la culture de carottes. Les larves infestantes dites de deuxième stade « J2 » se dirigent et pénètrent dans les racines pour migrer dans le cylindre central, former un syncytium et commencer à se développer, passent par les stades J3 et J4 pour parvenir jusqu'au stade adulte. La juvénile de troisième stade « J3 » se développe à l'intérieur de la cuticule du stade J2. Les juvéniles de troisième stade male apparaissent formés le quinzième jour après la pénétration des tissus alors que les femelles apparaissent entre le onzième et le seizième jour. Les juvéniles de stade 4, « J4 » montrent un dimorphisme sexuel marqué entre mâles et femelles. On notera que le développement au stade adulte passe par un déterminisme sexuel particulier, épigénétique, 25% des larves donnent des mâles, 25% donnent des femelles, les 50% restants déterminant leur sexe en fonction des conditions de ressources alimentaires disponibles, le sexe femelle étant choisi préférentiellement si les conditions alimentaires sont satisfaisantes. Les mâles filiformes vont quitter les racines alors que les femelles y restent fixées, apparaissant au travers des tissus déchirés, gonflées, remplies par les ovaires et étant fécondées dès qu'elles sont parvenues à maturité. On considère que la femelle adulte apparaît quelques 24 jours après la pénétration. Les femelles produisent deux types d'œuf, des œufs externes dans lesquels se forment des juvéniles de premier stade « J1 » qui maturent en larves de seconde génération « J2 » pour éclore rapidement, infestant à nouveau la culture et des œufs internes. Lorsque la femelle meurt, sa cuticule devient brune et se durcit, son corps formant un nouveau kyste abritant les œufs internes. Ce kyste pourra rester dans le sol une dizaine d'année en l'absence de nouvelle culture de carotte.
Le seuil d'apparition des dégâts sur la culture est généralement considéré comme atteint lorsqu'on constate la présence d'une larve J2 de nématode par gramme de sol.
La carotte est une espèce présentant une très grande variabilité, non seulement au sein de la carotte cultivée *Daucus carota* subsp. *sativus,* mais aussi au sein des douze sous espèces sauvages *Daucus carota.* La carotte sauvage est une plante spontanée dans de nombreuses régions du monde (Amérique, Afrique, Australie...) mais que l'on trouve principalement dans toute l'Europe et une bonne partie de l'Asie.
Les carottes cultivées appartiennent à la sous-espèce *sativus* (*Daucus carota* subsp. *sativus*)*.* Parmi celles-ci deux grands types sont généralement admis, les carottes de l'Est dites carottes asiatiques, rouge pourpre, violette (à anthocyanes) ou jaunâtres et les carottes à carotènes dites carottes de l'Ouest ou carottes européennes, de couleur orange, jaune ou blanche. Les carottes actuellement cultivées sont probablement originaires d'Afghanistan où elles sont connues dès le IXe siècle. A partir de ce centre primaire d'origine, leur extension se serait réalisée d'une part vers le Proche-Orient et la Chine, d'autre part vers le reste de l'Europe occidentale. La carotte a été introduite en France, à partir de l'Italie au XIVe siècle. Les carottes à carotènes apparaissent au XVIIe siècle aux Pays-Bas.
Les carottes sauvages se croisent assez facilement avec les carottes cultivées et donnant des hybrides fertiles. Toutefois si cette variabilité semble disponible, elle est loin d'être à ce jour exploitée car le retour vers une typologie de carotte cultivée, i.e. vers une plante *Daucus carota* subsp. *sativus* est extrêmement difficile et long.

Les inventeurs ont développé une stratégie inédite pour la désinfection ou désinfestation des sols contaminés par le nématode à kyste *Heterodera carotae,* permettant d'obtenir des taux de désinfection sensiblement identiques voire supérieurs à ceux obtenues par une désinfection chimique. Pour ce faire, les inventeurs ont développé de nouvelles plantes *Daucus carota,* résistantes au nématode à kyste *Heterodera carotae* ; ils ont par ailleurs démontré qu'en cultivant lesdites plantes, il était possible de tirer parti de leur résistance pour désinfecter un champ contaminé par *Heterodera carotae ;* en effet, de manière parfaitement originale, les inventeurs ont utilisé des plantes résistantes non pas en vue de les récolter, ni d'introgresser le trait lié à la résistance, mais pour restreindre la multiplication du nématode à kyste *Heterodera carotae,* c'est-à-dire qu'ils ont exploité l'effet nématicide sous tendant la résistance de ces plantes, qui par ailleurs sont non commerciales, c'est-à-dire qu'elles n'ont pas vocation à être récoltées pour être ensuite commercialisées à destination de l'alimentation, particulièrement l'alimentation humaine. Cette approche va à l'encontre des méthodes habituelles des sélectionneurs, qui visent généralement à identifier dans le germplasme sauvage des plantes résistantes à un nuisible donné et ensuite à introgresser cette résistance dans le patrimoine génétique de plantes commerciales, afin d'obtenir des variétés commerciales résistantes. Au contraire de ces méthodes, les présents inventeurs n'ont pas cherché à introgresser la résistance identifiée, mais à la stabiliser et à en tirer profit pour désinfecter des sols ou milieux de culture, selon les différentes utilisations ou méthodes de l'invention. De manière inattendue, les inventeurs ont ainsi mis en évidence que des plantes résistantes non commerciales, c'est-à-dire qui n'ont pas vocation à être récoltées pour être ensuite commercialisées à destination de l'alimentation notamment humaine, pouvaient être considérées comme des produits phytosanitaires non chimiques, permettant de désinfecter ou décontaminer des sols infestés par le nématode *Heterodera carotae.*
Les plantes résistantes obtenues par les inventeurs semblent en effet entraîner un blocage du cycle du nématode et ainsi, au fil du temps, elles diminuent l'infestation des champs. Sans vouloir être tenus par la théorie, il semblerait que les *Daucus carota* nématicides selon la présente invention aient une double action, non seulement au niveau du syncytium, en entrainant le blocage de son développement, mais aussi au niveau des mâles et des larves J2 de *Heterodera carotae* qui apparaissent bloqués au niveau des racines (nécroses).
Le déterminisme sexuel étant épigénétique chez le nématode à kyste *Heterodera carotae,* un blocage du syncytium va favoriser l'apparition de nématodes mâles au détriment de l'apparition de nématodes femelles. La population femelle diminuant fortement, la possibilité de former de nouveaux kystes au fils des générations va donc diminuer de manière similaire. De plus et de manière encore plus surprenante, les inventeurs ont constaté que les nématodes mâles et J2 semblent aussi bloqués dans la racine. S'ils ne peuvent sortir de la racine, ils ne peuvent donc féconder les femelles qui ne produiront pas d'œufs. Le cycle reproductif du nématode est là encore interrompu.
Ainsi, au fil du temps, les kystes infestant une parcelle de culture éclosent, du fait des exsudats racinaires produits par les plantes résistantes, et libèrent leurs larves qui sont ensuite bloquées dans leur cycle de multiplication, ne pouvant donc pas produire de nouveaux kystes. Sur plusieurs générations du nématode, par exemple sur une saison culturale, le niveau d'infestation de la parcelle de culture diminue à un niveau acceptable pour recevoir ensuite une culture de *Daucus carota sativus.*

### Définitions :

Par « désinfection » ou « désinfestation » d'un sol ou milieu, dans le cadre de l'invention, on entend la diminution de la population d'un ravageur ou pathogène, plus particulièrement d'un nématode, notamment un nématode à kystes, et plus spécifiquement *Heterodera carotae,* dans le sol ou milieu, ou bien la diminution de sa capacité de reproduction. Les termes désinfection et désinfestation vis-à-vis de *Heterodera carotae* sont utilisés de façon interchangeable dans la présente description.
Les notions de « résistance » et « sensibilité » sont définies d'une manière générale par l'ISF (International Seed Federation).
Ainsi, par « Résistance », on entend la capacité d'une plante ou d'une variété à restreindre la croissance et le développement d'un pathogène ou d'un ravageur déterminé et/ou les dommages qu'ils occasionnent, en comparaison avec des variétés sensibles et dans des conditions similaires, environnementales et de pression de ce pathogène ou de ce ravageur. Les plantes ou variétés résistantes peuvent exprimer quelques symptômes de la maladie ou quelques dommages en cas de forte pression du pathogène ou du ravageur. De préférence, dans le cadre de l'invention, la résistance s'entend de la capacité à restreindre la croissance et le développement d'un pathogène ou d'un ravageur déterminé.
Par « Sensibilité », on entend l'incapacité d'une plante ou d'une variété à restreindre la croissance et le développement d'un pathogène ou d'un ravageur déterminé.
Dans la cadre de l'invention, la résistance d'une plante au nématode *Heterodera carotae* s'entend de la capacité de cette plante à restreindre la croissance et le développement dudit nématode. Une plante sensible à ce même nématode est par exemple la variété de carotte Nanco, commercialisé par la société Vilmorin.
Une plante sera considérée comme résistante lorsqu'elle présente une absence de multiplication de nématodes et/ou une réduction de la multiplication de nématode accompagnée éventuellement de la production d'individus mâles de façon très majoritaire. Dans le premier cas, les nématodes sont bloqués au niveau de l'initiation du syncytium nourricier: les larves ne parviennent pas à "détourner" le métabolisme cellulaire a leur profit et ne peuvent donc accomplir leur cycle. Dans le second cas, certaines larves parviennent à initier puis à entretenir un syncytium nourricier de mauvaise qualité et évolueront donc plutôt vers des mâles, moins exigeants en apports énergétiques.
Par « Effet nématicide d'une plante » ou action nématicide ou pouvoir nématicide d'une plante, on entend principalement la capacité de cette plante à réduire la population d'un nématode, présent dans un milieu, ou bien la capacité de réduire la population correspondant à un stade de développement dudit nématode, par exemple réduction du nombre de larves au stade J2, ou bien la réduction du nombre de kystes. L'effet nématicide selon la présente invention est spécifiquement dirigé vers le nématode à kyste *Heterodera carotae.* L'effet ou pouvoir nématicide s'entend également de la capacité de masculinisation à plus de 50 % des larves du nématode. Une plante ayant un effet nématicide est une plante qui donc restreint la croissance ou le développement du nématode par rapport à une plante *Daucus carota* subsp. sativus, dans des conditions de culture similaires. Il est à noter que l'effet nématicide selon la présente invention ne provient pas de la méthode culturale en tant que telle, mais bien de la capacité de la plante à restreindre la multiplication et le développement du nématode, contrairement à la culture de carottes-pièges, où la carotte ne sert qu'à attirer et canaliser les nématodes pour ensuite plus facilement les éliminer en arrachant la carotte. L'effet ou pouvoir nématicide selon l'invention est donc obtenu grâce à un mécanisme de résistance desdites plantes à l'encontre du nématode à kyste *Heterodera carotae.*
L'effet ou pouvoir nématicide d'une plante à l'encontre de *Heterodera carotae* peut par exemple être testé in vitro comme décrit à l'exemple 1B, par inoculation de larves de *Heterodera carotae* directement sur l'apex de graines germées et le comptage du nombre de larves ou de la proportion femelles/males, après 15 à 20 jours.
De préférence, le pouvoir nématicide d'une plante est mis en évidence en semant des graines dans un pot rempli de terre présentant un taux moyen de 15 larves par gramme de sol ; une plante sera considérée comme résistante à *Heterodera carotae,* ou bien comme possédant une pouvoir nématicide à l'encontre de *Heterodera carotae,* si 90 jours après le semis, elle présente moins de 10 femelles, larves et/ou kystes, au niveau des racines. La mise en œuvre de ce test est détaillée notamment dans l'exemple 2 de la partie expérimentale.
Au niveau d'une population, une population de plantes est considérée comme globalement résistante à *Heterodera carotae,* ou présentant globalement un pouvoir nématicide à l'encontre de *Heterodera carotae,* si au moins 70% des plantes constituant la population sont résistantes ou possèdent un pouvoir nématicide comme défini plus haut, de préférence au moins 75%, de façon toute préférée au moins 80%. Une population de plantes résistante ou présentant un pouvoir nématicide ne comprend de préférence aucune plante multiplicatrice, notamment aucune plante présentant plus de 100 larves au niveau des racines, 90 jours après le semis dans un sol comprenant en moyenne 15 larves ou kystes par gramme de sol. Par « diminuer suffisamment le niveau d'infestation » d'un champ de culture par le nématode à kyste *Heterodera carotae* afin de permettre une culture de carotte, on entend abaisser le niveau d'infestation d'*Heterodera carotae* en deçà du nombre de larves J2 entraînant l'apparition de dégâts sur la culture. Ce seuil est généralement d'une larve J2 de nématode par gramme de sol ou bien d'un kyste de nématode par gramme de sol.

Selon un premier aspect, la présente invention concerne donc l'utilisation d'une plante de l'espèce *Daucus carota,* pour traiter un sol ou un milieu de culture, infesté ou susceptible d'être infesté par des nématodes, plus spécifiquement des nématodes à kyste, et tout préférentiellement *Heterodera carotae,* en vue de le désinfecter. La plante utilisée selon l'invention est une plante présentant un effet ou pouvoir nématicide, notamment à l'encontre du nématode *Heterodera carotae.* Selon une mise en œuvre particulièrement préférée de l'invention, ladite plante est une carotte *Daucus carota* qui n'appartienne pas à la sous-espèce des carottes cultivées *Daucus carota* subsp. *sativus.*
Par effet nématicide de la plante, conformément aux définitions données ci-dessus, on entend en particulier la capacité de réduire le nombre de larves juvéniles J2 infestant un milieu, ou bien la capacité de réduire le nombre de kystes, ou bien encore la capacité d'entrainer une masculinisation supérieure à 50 % des larves du nématode *Heterodera carotae* infestant un milieu. Il peut s'agir également d'une combinaison de ces propriétés, par exemple la capacité à réduire le nombre de larves J2 et le nombre de kystes, ou bien la capacité de réduire le nombre de J2 et d'entrainer une masculinisation ; ou bien encore de la combinaison de ces trois propriétés.
De préférence, la masculinisation engendrée par l'utilisation selon cet aspect de l'invention est une masculinisation supérieure à 60%, de préférence supérieure à 70%, voire à 80% de la population de larves *Heterodera carotae* infestantes.
Il est à noter que le pouvoir ou effet nématicide est propre à la plante ou graine, il n'est pas le résultat de la méthode de culture ; en particulier, il ne provient pas d'un arrachage précoce des plantes ou bien d'une culture dans des conditions d'arrosage insuffisant, qui sont connus pour engendrer une diminution de la population de nématodes infestant un sol ou bien un milieu de culture.
Cette capacité nématicide est observée de préférence lors d'un temps de culture desdites carottes d'au moins 3 mois, mais de préférence d'au moins 4 mois, par exemple de 5 mois, ou bien de 6 mois, ou bien plus de 6 mois.
Comme mentionné, selon une mise en œuvre particulièrement préférée de l'invention, la plante utilisée est une carotte *Daucus carota* qui n'appartienne pas à la sous-espèce *Daucus carota* subsp. *sativus* ; la carotte utilisée pour la désinfection n'est donc pas une carotte commerciale, elle n'a pas vocation à être récoltée pour la commercialisation à destination de l'alimentation, notamment humaine, et n'est donc pas destinée à la consommation. La carotte nématicide utilisée dans le cadre de la présente invention est à considérer comme un produit de désinfection, ou produit phytosanitaire, utilisée pour désinfecter un sol ou milieu de culture infesté de nématodes *Heterodera carotae* ou susceptible de l'être.
Il peut s'agir notamment d'une plante appartenant à la sous-espèce *Daucus carota dentatus,* ou bien une plante issue de cette sous-espèce ou d'une autre sous-espèce sauvage. Il peut s'agir notamment d'une plante hybride dont l'un au moins des deux parents est une carotte dite sauvage, qui n'appartient pas à la sous-espèce cultivée *Daucus carota* subsp. *sativus* ; par exemple il s'agit d'une plante nématicide dont l'un au moins des deux parents est une carotte *Daucus carota dentatus.*

Selon une mise en œuvre tout particulièrement préférée, les carottes utilisées dans le cadre de cette utilisation sont des plantes issues de graines déposées au NCIMB sous le numéro d'accession NCIMB 42351.
Les présents inventeurs ont effet obtenu des plantes présentant une très forte résistance au nématode à kyste *Heterodera carotae,* comme illustré dans la partie expérimentale. Lesdites plantes présentent une double action nématicide, non seulement au niveau du développement du syncytium mais aussi au niveau des mâles et des larves J2 qui apparaissent bloqués au niveau des racines (nécroses). Chez *Heterodera carotae,* un blocage du syncytium favorise l'apparition de nématodes mâles au détriment de l'apparition de nématodes femelles. La population femelle diminuant fortement, la possibilité de former de nouveaux kystes au fil des générations diminue donc de manière similaire. De plus et de manière encore plus surprenante, les inventeurs ont constaté que les nématodes mâles et J2 semblent aussi bloqués dans la racine (nécroses). S'ils ne peuvent sortir de la racine, ils ne peuvent donc féconder les femelles qui ne produiront pas d'œufs. Le cycle est là encore interrompu, d'où la double action nématicide des plantes telles que décrites dans la partie expérimentale.
Des graines permettant d'obtenir des plantes *Daucus carota* possédant lesdites propriétés nématicides ont été déposées par la société Vilmorin, Route Le Manoir, 49250 La Ménitré, France, conformément aux exigences du Traité de Budapest pour la reconnaissance internationale du dépôt des micro-organismes aux fins de la procédure en matière de brevets, le 20 janvier 2015, auprès du « National Collection of Industrial, Food and Marine Bacteria » (NCIMB), (NCIMB Ltd, Ferguson Building, Craibstone Estate, Bucksburn, Aberdeen AB21 9YA, Royaume-Uni), sous le numéro d'accession NCIMB 42351, avec la référence Daucus carota DCHR1.
Selon une mise en œuvre particulièrement préférée, les plantes utilisées pour la désinfection selon l'invention sont des plantes développées à partir de graines NCIMB 42351 ou bien des plantes issues ou dérivées de ces graines, après propagation ou bien croisement et sélection. Les plantes utilisées peuvent notamment être des plantes *Daucus carota* dérivées de plantes provenant des graines déposées sous le numéro NCIMB 42351. La présente description fournit toutes les informations nécessaires pour sélectionner les plantes possédant le pouvoir nématicide, dans le cadre d'un programme de sélection.
La demande n'est par ailleurs nullement limitée aux plantes issues des graines telles que déposées. En effet, comme décrit dans l'exemple 1 de la demande, de nombreux génotypes sauvages sont susceptibles de présenter également une résistance au nématode *Heterodera carotae,* résistance qu'il est possible d'utiliser, selon l'enseignement de la présente invention, pour désinfecter un sol ou milieu de culture.
Les plantes utilisées dans le cadre de l'invention peuvent être une population de plantes nématicides, c'est-à-dire qu'au moins 70% des plantes de ladite population possèdent en effet ce pouvoir nématicide, et de préférence au moins 80 à 90% des plantes, voire plus. Il n'est donc pas exclu que certaines plantes, au sein de la population, ne possèdent pas, individuellement, de pouvoir nématicide propre. De préférence, la population ne comprend toutefois qu'un très faible pourcentage, inférieur à 5%, ou inférieur à 2 ou 1%, de plantes multiplicatrices du nématode.
Le sol ou milieu de culture infesté, ou susceptible d'être infesté, peut être tout milieu convenant à la culture de carottes. Il peut s'agir notamment de terre, ou bien de sable, ou bien d'un terrain comprenant un mélange terre et sable. Un milieu de culture est considéré comme infesté s'il comprend des kystes de nématode *Heterodera carotae,* ou bien s'il comprend ces nématodes à un quelconque autre stade de développement, par exemple s'il comprend des juvéniles J2. On considère généralement qu'un champ ou un milieu de culture est infesté quand ledit champ ou milieu de culture comprend au moins une larve J2 de nématode par gramme de sol, ou bien un kyste. Au-delà de ce seuil, il est en effet considéré qu'il est nécessaire de le désinfecter avant de cultiver des carottes commerciales, destinées à l'alimentation.
Cependant l'utilisation selon la présente invention est également applicable sur tout sol ou milieu de culture qui est susceptible d'être infesté. Il est par exemple envisageable d'opérer un cycle de désinfection avec des plantes nématicides dans le cadre d'un plan de rotation des cultures. Il est également envisageable de procéder à une telle désinfection systématiquement avant toute culture de carottes commerciales, ou bien en vue de commercialiser le milieu de culture lui-même.
Par la mise en œuvre de l'utilisation telle que décrite plus haut, il est ainsi possible de procéder à une désinfection ou désinfestation d'un milieu de culture infesté par le nématode, et ainsi de réduire la population de nématodes au sein dudit milieu de culture d'au moins 40 %, de préférence d'au moins 50%, de façon encore plus préférée d'au moins 60 % et tout particulièrement d'au moins 75%. Il est à noter cependant que le niveau de réduction de la population de nématodes opérée par l'utilisation de plantes nématicides conformément à la présente invention, est fonction du niveau d'infestation initial du milieu de culture, du temps de culture des plantes nématicides, et également de la densité des plantes nématicides.
De préférence, comme détaillé précédemment, les plantes nématicides selon l'invention sont cultivées durant un temps de culture d'au moins 3 mois, afin de générer le plus grand pouvoir nématicide, et plutôt au moins quatre mois, voire cinq mois ou bien six mois. Il est important de noter que ce temps de culture se distingue du temps de culture généralement retenu dans le cadre d'utilisation de carottes pièges. En effet, dans une telle situation, la carotte cultivée doit être retirée du sol une fois qu'elle a déclenché l'éclosion des kystes présents dans le milieu de culture, mais avant la formation de nouveaux kystes ou la sortie de nombreux nématodes mâles des racines. L'utilisation de plantes nématicides telles que décrites dans le cadre de l'invention est au contraire d'autant plus efficace que la carotte nématicide reste longtemps dans le milieu, de préférence jusqu'à complète maturation des racines, sur plusieurs générations du nématode.
De même, les plantes nématicides sont plantées à une densité de graines comparable ou supérieure à celle utilisée pour des variétés de carottes, cultivées pour la consommation. Des densités particulièrement appropriées dans le cadre de l'invention sont notamment celles comprises entre 0,8 et 4 millions de graines par hectare, et de préférence entre 0,8 et 2,5 millions de graines par hectare. De telles densités permettent en effet de générer une désinfection optimale du milieu de culture.
Selon une mise en œuvre préférée de l'invention, l'utilisation de plantes nématicides telles que décrites permet de réduire le nombre de kystes ou bien de nématodes femelles du milieu de culture d'au moins 50 %. En effet, des plantes nématicides selon la présente invention peuvent également engendrer une importante masculinisation de la population des larves, sans entrainer nécessairement une réduction de leur nombre, du moins dans un premier temps. Cependant, la réduction du nombre de femelles au profit de larves mâles entraine ensuite une diminution importante du nombre de kystes au sein du milieu de culture.
De préférence, l'utilisation telle que décrite permet de réduire le niveau d'infestation du milieu de culture à moins d'une larve J2 femelle d'*Heterodera carotae* par gramme de milieu ou à moins d'un kyste d'*Heterodera carotae* par gramme de milieu. Une telle réduction s'entend bien entendu d'un milieu de culture comprenant initialement, avant la mise en œuvre de l'utilisation selon l'invention, d'un milieu de culture comprenant plus d'une larve J2 femelle ou plus d'un kyste d'*Heterodera carotae* par gramme de milieu, par exemple au moins cinq larves J2 femelles et/ou kystes d'*Heterodera carotae* par gramme de milieu, par exemple environ 10 larves J2 et/ou kystes par gramme de milieu, ou même plus de 10 larves J2 et/ou kystes par gramme. Des sols infestés peuvent en effet contenir plus de 30 ou même plus de 50 larves J2 et/ou kystes par gramme.
Des carottes issues des graines déposées sous le numéro NCIMB 42351 permettent notamment d'obtenir les mises en œuvre préférées décrites ci-dessus, et tout particulièrement la réduction du nombre de larves juvéniles J2 et/ou la réduction du nombre de kystes et/ou la masculinisation supérieure à 50 % des larves du nématode *Heterodera carotae* lors d'une culture d'au moins 4 mois, ainsi que la réduction de la population de nématodes *Heterodera carotae* au sein du milieu de culture d'au moins 60%, tout préférentiellement d'au moins 75%, la réduction d'au moins 50% du nombre de kystes ou de nématodes femelles du milieu de culture, ainsi que la réduction du niveau d'infestation du milieu de culture à moins d'une larve J2 femelle d'*Heterodera carotae* par gramme de milieu comme décrit ci-dessus. De telles plantes sont par exemple des plantes obtenues par germination de graines déposées NCIMB 42351, ou bien par sélection de plantes ayant les propriétés attendues, à partir de plantes issues des graines déposées, après un ou plusieurs croisements.

L'utilisation des plantes nématicides telles que décrites dans la présente invention permet d'assurer une désinfection qui non seulement convienne d'un point de vue environnemental et écologique, mais qui de plus soit reproductible, certaine et facile à mettre en œuvre. En effet, l'utilisation par exemple de carottes pièges permet difficilement une désinfection reproductible. Par ailleurs, si dans certaines conditions, des cultures de carottes commerciales peuvent diminuer le niveau d'infestation (voir exemple 9), cette diminution n'est nullement reproductible (voir exemple 7, avec la même variété commerciale).

Selon un second aspect, la présente invention concerne également une méthode permettant de désinfecter un sol ou milieu de culture, ou bien de réduire la population de nématodes, plus particulièrement de nématodes à kystes, spécifiquement *Heterodera carotae* dans un sol ou milieu de culture infesté, ou susceptible d'être infesté par le nématode à kyste *Heterodera carotae.* Ladite méthode de désinfection ou désinfestation se base, de façon originale, sur la culture de plantes *Daucus carota,* où lesdites plantes présentent un effet nématicide sur ces nématodes.
Selon une mise en œuvre tout particulièrement préférée, ladite méthode de désinfection ne comprend pas d'étape d'application de produit chimique à effet nématicide, tel que le 1,3 dichloropropène ou ses dérivés, sous quelque forme que ce soit. Par conséquent, la désinfection n'est que le résultat de l'action nématicide des plantes cultivées.
Selon une autre mise en œuvre, la méthode de désinfection ou désinfestation selon l'invention peut être accompagnée, précédée, suivie ou complétée par l'application d'un produit chimique nématicide. Dans ce cas, l'étape principale de culture de plantes nématicides permet de réduire la dose de produit chimique nématicide qu'il aurait été nécessaire d'utiliser pour obtenir un niveau de désinfection comparable, en l'absence de culture de plantes nématicides. Dans le cadre de cette mise en œuvre, la culture de plantes selon l'invention et l'application de produit chimique nématicide peuvent être concomitantes, successives ou bien se chevaucher totalement ou partiellement ; l'ordre des étapes n'est pas déterminant.
La réduction de la population de nématodes *Heterodera carotae* dans un sol ou milieu de culture infesté, ou susceptible d'être infesté par le nématode, s'entend de préférence d'une réduction du nombre de kystes dudit nématode, au sein du sol ou milieu de culture, ou bien d'une réduction de juvéniles J2, ou bien cumulativement d'une réduction à la fois du nombre de kystes et du nombre de juvénile J2 au sein du milieu de culture.
De préférence, les plantes cultivées dans le cadre de cette méthode de désinfection n'appartiennent pas à la sous-espèce des carottes cultivées *Daucus carota* subsp. *sativus.*
Les mises en œuvre préférées détaillées dans le cadre des différentes utilisations selon le premier aspect de l'invention sont également applicables aux méthodes selon ce second aspect de l'invention. Notamment, la méthode est de préférence mise en œuvre avec des plantes nématicides de la sous-espèce *Daucus carota dentatus,* ou bien des plantes dérivant de carottes de la sous-espèce *Daucus carota dentatus,* par exemple issues d'un croisement initial entre une plante *Daucus carota dentatus* et une plante *Daucus carota sativus.*
Comme pour ce qui concerne les différentes utilisations de l'invention, les méthodes sont également de préférence mises en œuvre avec des carottes qui sont issues ou dérivées des graines déposées par Vilmorin auprès du NCIMB le 20 janvier 2015, sous le numéro d'accession NCIMB 42351. De telles plantes sont par exemple obtenues par germination directe desdites graines déposées. Il peut s'agir également de plantes qui sont obtenues par propagation de carottes provenant de la germination des graines déposées auprès du NCIMB, ou bien obtenues par des croisements avec d'autres plantes, de préférence de l'espèce *Daucus carota.* De telles plantes sont décrites dans la partie expérimentale de la présente demande. Des plantes nématicides utilisables dans les méthodes selon l'invention sont également des plantes régénérées, éventuellement *in vitro,* à partir de cellules de plantes obtenues par germination des graines déposées sous le numéro NCIMB 42351. Cependant, les méthodes de l'invention ne sont nullement limitées aux plantes nématicides dérivées des graines déposées sous le numéro d'accession, d'autres plantes *Daucus carota* ayant un pouvoir nématicide peuvent être utilisées, qui ne soient pas *Daucus carota sativus,* et qui sont identifiables notamment par la mise en œuvre des tests décrits dans les exemples de la partie expérimentale.
Comme déjà mentionné, les plantes utilisées dans le cadre de l'invention peuvent être une population de plantes nématicides.
Le sol ou milieu de culture infesté, ou susceptible d'être infesté, est comme décrit pour les utilisations selon la présente invention. Il s'agit de préférence d'un champ, il peut s'agir de terre ou de sable ou d'un mélange des deux, ou bien d'un terrain sablonneux.
Par la mise en œuvre de la méthode de l'invention, il est ainsi possible de procéder à une désinfection ou désinfestation d'un sol ou milieu de culture, infesté par le nématode, et ainsi de réduire la population de nématodes au sein dudit milieu de culture d'au moins 40 %, de préférence d'au moins 50%, de façon encore plus préférée d'au moins 60 % et tout particulièrement d'au moins 75%. Par cette méthode, il est donc possible de réduire le niveau d'infestation du sol ou milieu de culture à moins d'une larve J2 femelle et/ou kyste d'*Heterodera carotae* par gramme de milieu, c'est-à-dire en deçà du seuil correspondant à un milieu dit « infesté ». La méthode est de préférence mise en œuvre sur un milieu de culture comprenant plus d'une larve J2 et/ou kystes par gramme de milieu, de préférence au moins 5 larves J2 et/ou kystes par gramme de milieu, ou bien environ 10 ou plus de 10 larves J2 et/ou kystes par gramme de milieu, voire 20, 25, 50 ou plus.
Selon une mise en œuvre préférée de l'invention, la méthode permet de réduire le nombre de kystes ou bien de nématodes femelles du milieu de culture d'au moins 50 %. La réduction du nombre de femelles peut être due à une réduction globale du nombre de nématodes, ou bien à une proportion diminuée de femelles par rapport aux mâles. En effet, des plantes nématicides selon la présente invention peuvent engendrer une importante masculinisation de la population des larves, sans entrainer forcément une réduction immédiate de leur nombre, du moins dans un premier temps. Cependant, la réduction du nombre de femelles au profit de larves mâles entraine ensuite une diminution drastique du nombre de kystes au sein du sol ou milieu de culture.
La méthode selon l'invention comprend la culture de plantes nématicides durant au moins 3 moins et de préférence pour une durée d'au moins 4 mois, de préférence d'au moins 5 mois, tout préférentiellement d'au moins 6 mois. Comme relevé en regard des utilisations selon l'invention, ce temps de culture est très différent des temps de culture applicables dans le cadre d'une culture de carottes-pièges, où le maintien des carottes pièges au-delà de 3 mois conduit à multiplier le nombre de kystes ou larves infestantes au lieu de le diminuer. La méthode selon l'invention est au contraire bien plus aisée à mettre en œuvre dans la mesure où les carottes nématicides peuvent être retirées à tout moment, de préférence après 4 mois, sans aucun effet préjudiciable sur le niveau d'infestation. En effet, quel que soit le moment où elles sont retirées, de préférence toutefois après 3 mois, on obtiendra toujours une diminution du niveau d'infestation du milieu de culture. L'effet nématicide optimal est obtenu quand environ un temps de culture correspondant à cycle de culture complet pour une variété commerciale de carotte *D. carotae* est réalisé, soit environ 6 mois. Cependant, un bon effet nématicide est déjà observé après 3 mois de culture, de préférence après 4 mois. Le moment d'arrachage optimal, tenant compte d'une part de l'absence de production commerciale durant le temps de culture des carottes nématicides, et d'autre part de l'effet nématicide optimal, semble se situer entre 4 et 6 mois. Bien entendu, ce moment optimal doit être déterminé en tenant également compte de la probabilité d'infestation du milieu de culture, et de son niveau d'infestation.
Selon la présente méthode, les plantes nématicides sont plantées à une densité de graines comparable ou supérieure à celle utilisée pour des carottes dites commerciales, cultivées pour la consommation. Des densités particulièrement appropriées dans le cadre de l'invention, lorsqu'elle est mise en œuvre dans des champs, sont notamment celles comprises entre 0,8 et 4 millions de graines par hectare, et de préférence entre 0,8 et 2,5 millions de graines par hectare. De telles densités permettent en effet de générer une désinfection optimale du milieu de culture. D'autres densités sont toutefois envisageables, notamment quand il s'agit de petites quantités de milieu de culture, ou bien quand le niveau d'infestation est particulièrement fort ou bien particulièrement faible. La densité peut également être adaptée en fonction de la composition du milieu de culture.
Lors de la mise en œuvre de l'invention, la culture de carottes nématicides peut être initiée à tout moment opportun, en fonction des cultures précédant et suivant la culture de carottes nématicides. Selon des mises en œuvre préférées, ladite culture est par exemple initiée à l'automne ou à l'hiver, afin de fournir le meilleur rendement nématicide et/ou le plus faible impact sur la rentabilité. En fonction du climat, ou d'autres contraintes, par exemple économiques, la culture peut cependant être initiée à d'autres moments si cela s'avère approprié, nécessaire ou souhaitable. La culture est de préférence initiée en semant des graines, bien qu'il puisse également être envisagé de repiquer des plants.
La méthode selon la présente invention permet d'obtenir une diminution de la population de nématodes, en particulier femelles, d'*Heterodera carotae* dans un milieu de culture infesté essentiellement grâce aux propriétés nématicides des plantes cultivées. Comme détaillé plus haut, cette caractéristique de la méthode la distingue notamment de la culture de carottes pièges, où la diminution de la population de nématodes tient à la méthode de culture, et plus particulièrement au moment d'arrachage, mais pas aux plantes cultivées.
Certaines mesures peuvent cependant être prises dans le cadre de l'invention afin d'amplifier la réduction de la population de nématodes ; notamment, certaines conditions de culture ou bien certains engrais ou autres, peuvent être appliqués afin d'amplifier l'effet nématicide engendré par les plantes nématicides selon l'invention. Notamment, les plantes peuvent éventuellement être cultivées dans des conditions d'hygrométrie insuffisante, dans la mesure où une certaine sécheresse du milieu de culture est connue pour participer à la réduction de la population de nématodes *Heterodera carotae.*
Alternativement, la méthode peut être réalisée dans des conditions d'hygrométrie satisfaisantes pour une culture de carottes commerciales.
Des carottes issues des graines déposées sous le numéro NCIMB 42351 permettent notamment d'obtenir les mises en œuvre préférées décrites ci-dessus, pour les diverses méthodes selon l'invention, notamment des plantes obtenues par germination des graines déposées.

Selon un autre aspect, la présente description divulgue également une population de plantes *Daucus carota,* ou bien une population de graines de carottes *Daucus carota,* provenant ou issues de graines déposées sous le numéro d'accession NCIMB 42351 où ladite population ou lesdites carottes présentent un effet nématicide sur des nématodes, notamment de nématodes à kystes, et spécifiquement *Heterodera carotae.* L'effet nématicide est tel que décrit dans la présente demande. Des plantes provenant des graines déposées sont notamment des plantes obtenues par germination desdites graines. Des plantes ou graines provenant ou issues de graines déposées couvrent aussi des plantes ou graines obtenues par croisement ou propagation de plantes obtenues par germination des plantes déposées, ainsi que toutes les plantes dérivées de plantes obtenues par germination des graines déposées, à condition toutefois qu'elles possèdent un pouvoir nématicide, et notamment le pouvoir nématicide tel que décrit pour les plantes correspondant aux graines déposées et illustré dans la partie expérimentale de la demande.

La présente description divulgue aussi des fragments de ces plantes, des semences, du matériel de reproduction et des cellules de ces plantes, qui peuvent être régénérés pour obtenir des plantes résistantes, ou nématicides.
Il a déjà été décrit dans le cadre des précédents aspects de l'invention les mises en œuvre préférées de l'invention ; elles sont également applicables à cet aspect de la description. Notamment, l'effet nématicide des plantes est de préférence une réduction du nombre de juvéniles J2 et/ou une réduction du nombre de kystes et/ou une masculinisation supérieure à 50 % des larves du nématode *Heterodera carotae,* de préférence lors d'un temps de culture desdites carottes d'au moins 3 mois.
Ladite population comprend au moins 70% de plantes présentant individuellement un pouvoir nématicide tel que décrit, ou bien 70% de graines donnant lieu à de telles plantes. De préférence, la proportion est supérieure à 70%, par exemple au moins 80%, voire au moins 90% ou plus.
De préférence, la population ne comprend toutefois qu'un très faible pourcentage, inférieur à 5%, ou inférieur à 2 ou 1%, de plantes multiplicatrices du nématode, ou de graines donnant lieu à de telles plantes.

Comme détaillé précédemment, la présente invention est illustrée par les plantes *Daucus carota* identifiées par les inventeurs et présentant une résistance au nématode, notamment au nématode à kyste *Heterodera carotae,* cependant, l'invention n'est pas limitée au génotype identifié par les inventeurs. En effet, d'autres plantes présentant également un pouvoir nématicide peuvent aisément être identifiées par l'homme du métier. Dans ce but, un test adéquat pour déterminer le pouvoir nématicide d'une plante ou d'une population, ou sélectionner les plantes possédant un tel pouvoir est le suivant :
- Des graines à tester sont semées dans un pot rempli de terre infestée à environ 10 à 15 larves d'*Heterodera carotae* par gramme de sol ;
- La culture est poursuivie durant 70 à 90 jours dans des conditions normales de culture ;
- 70 à 90 jours après le semis, les plantes sont arrachées et le nombre de femelles J2 au niveau des racines est compté.
Une plante possédant un pouvoir nématicide selon l'invention est de préférence une plante présentant entre 0 et 10 femelles au niveau des racines à l'issue de ce test. A l'inverse, une plante présentant plus de 100 femelles au niveau des racines sera considérée comme une plante multiplicatrice.
Par ce protocole, il est aisé d'identifier des plantes nématicides, ou de sélectionner des plantes nématicides, notamment dans un programme de sélection.
La présente description divulgue également sur un produit phytosanitaire, permettant la désinfection ou désinfestation d'un sol ou milieu de culture, ledit produit comprenant des plantes nématicides ou résistantes, telles que décrites. Toutes les caractéristiques déjà décrites précédemment sont applicable à cet aspect de la divulgation. Notamment, un produit phytosanitaire tel que mentionné peut comprendre une population de plantes ou de graines D. carota, et de préférence qui ne soit pas de la sous-espèce Daucus carota sativus, au moins 70% d'entre elles présentant un effet ou pouvoir nématicide.
Il est particulièrement avantageux que le produit phytosanitaire ne comprenne pas de plantes multiplicatrices, telles que définies plus haut.
La présente invention se caractérise donc entre autres par le fait de cultiver ou utiliser des plantes *D. carota* non commerciales, c'est-à-dire qu'elles n'ont pas vocation à être récoltées à destination de l'alimentation, particulièrement l'alimentation humaine, et d'exploiter la résistance de ces plantes à *Heterodera carotae* pour obtenir un effet nématicide, permettant de désinfecter un champ infesté par ce nématode.

### Légende des figures

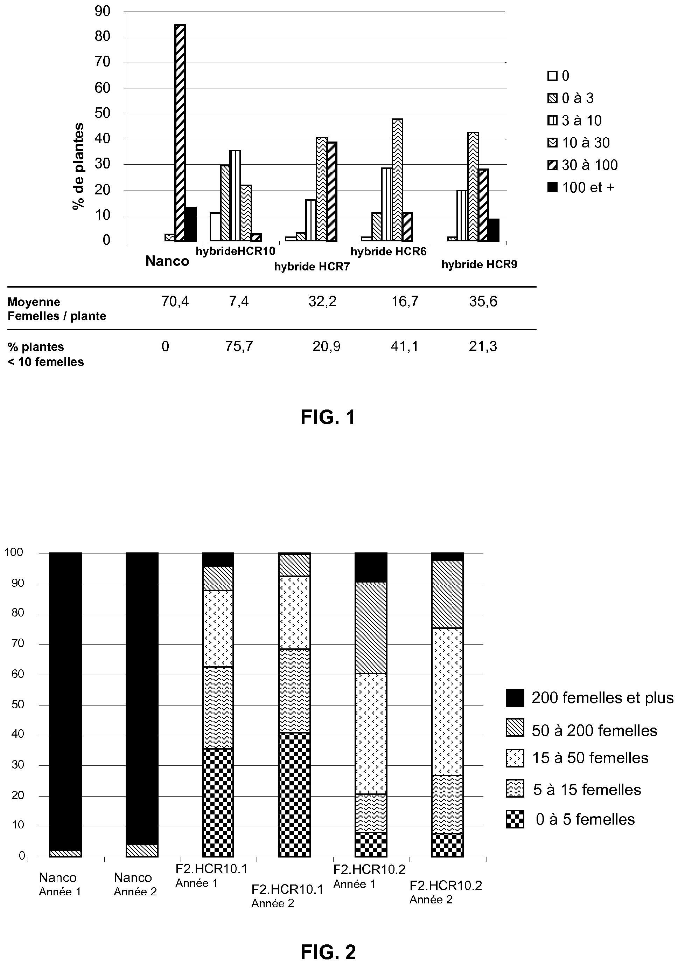

**Fig.1** : Le graphique illustre, pour différentes combinaisons hybrides issues des génotypes identifiés à l'exemple 1, ainsi que pour la variété sensible Nanco, la distribution du nombre de femelles *Heterodera carotae* par plante, le nombre moyen de femelles par plantes, et le pourcentage de plantes présentant moins de 10 femelles *Heterodera carotae.*
**Fig. 2** : Le tableau indique, pour deux lignées F2 issues du génotype HCR10, la distribution du nombre de femelles en moyenne, par plante, sur deux années de culture.

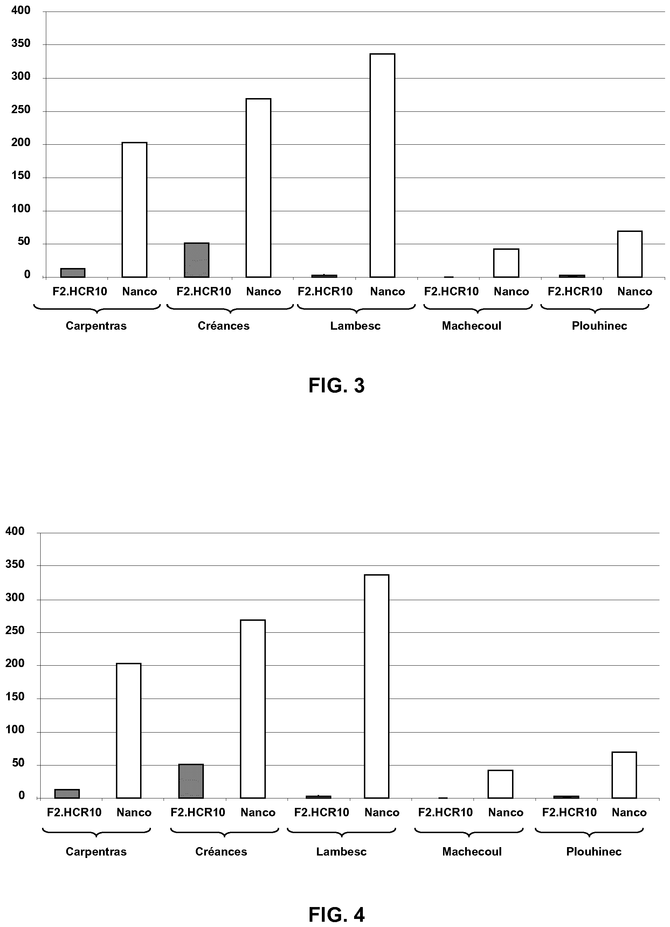

**Fig. 3** et **4** : Ces figures décrivent le nombre moyen de femelles présentes dans les racines, environ 80 à 90 jours après le semis, dans des milieux de culture infestés prélevés dans 5 zones géographiques distinctes, dans des champs de cultures infestés par *Heterodera carotae* à Créance dans la Manche, à Plouhinec dans le Morbihan, à Machecoul en Vendée, à Carpentras dans le Vaucluse et à Lambesc dans les bouches du Rhône. Leur niveau de contamination varie de 4,46 à 13,86 larves J2 par gramme de sol.
La figure 3 concerne des semis du 3 mai avec une lecture au 19 juillet alors que figure 4 concerne des semis du 28 mai avec une lecture au 24 aout.

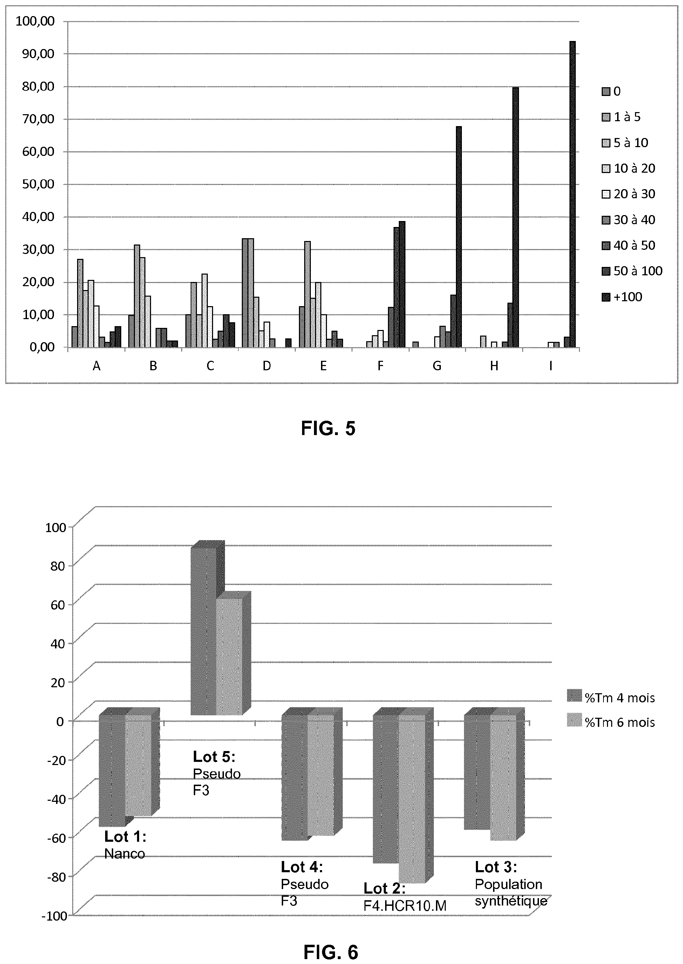

**Fig. 5** : La figure 5 illustre les résultats présentés dans le tableau 2. En abscisse, on retrouve les différents génotypes A à I testés. En ordonnée est porté le pourcentage de plantes, au sein d'un génotype, appartenant à chacune des 9 classes suivantes : 0 ; 1 à 5 femelles; 5 à 10 femelles; 10 à 20 femelles; 20 à 30 femelles; 30 à 40 femelles; 40 à 50 femelles; 50 à 100 femelles et 100 à 1000 femelles.
**Fig. 6** : La figure 6 illustre le taux de multiplication des kystes dans le sol de jardinières, où sont mis en culture différents lots de graines, sensibles et résistantes, après 4 mois et 6 mois de culture. Le taux d'infestation au moment du semis est identique dans chaque jardinière. %Tm signifie : taux de multiplication des kystes dans le sol.

### Partie expérimentale

### Exemple 1 : Identification de plantes résistantes à Heterodera carotae

Les inventeurs ont procédé en trois étapes successives.

Dans un premier temps, ils ont testé plus de 3700 plantes différentes, d'origines génétiques et géographiques diverses au moyen d'un test d'inoculation racinaire. Seules les plantes ne présentant pas de nématode *Heterodera carotae* femelle, particulièrement ne présentant aucun kyste, ont été retenues pour la seconde analyse (test de confirmation de la résistance in vivo).

### A. Obtention des juvéniles (J2) Heterodera carotae

Les kystes sont récoltés dans des parcelles infectées en basse Normandie et multipliés en serres avant d'être conservés à 4°C jusqu'à leur utilisation. Les kystes sont ensuite humidifiés dans de l'eau, pendant 24 h, avant d'être transférés dans de l'exsudat radiculaire de carotte et déposés en chambre climatisée à 20°C. Les larves, âgées au maximum d'une semaine, sont recueillies et conservées à 4°C dans de l'eau.

### B. Décontamination des graines de carotte et inoculation des larves de Heterodera carotae (Etape 1)

La décontamination des graines de carotte s'effectue à 52°C au bain-marie pendant 18 minutes dans de l'eau stérile additionne de SDS 10%. Les graines sont ensuite rincées dans de l'eau stérile et déposées sur milieu gélosé à 1.5%.

Les graines ayant germées sont transférées individuellement dans une boite de Petri sur milieu gélosé additionné de micro éléments, milieu MS (Murashige T and Skoog F (1962)). Les juvéniles *Heterodera carotae* de stade J2 sont inoculées sur les plantules dont la racine atteint de 1 à 2 cm de long.

Sept J2 sont déposées sur l'apex de la racine à l'aide d'un cil. Les boites sont laissées 48 h au laboratoire avant d'être déposées en chambre climatisée à 20°C avec une photopériode de 16 h.

Pour chaque génotype (plante) testé, 60 graines sont mises à germer. Le nombre de plantes réellement évaluées (inoculées) pour leur résistance dépend ensuite du taux de germination obtenu dans les boites de Petri.

Un hybride commercial *Daucus Carota sativus* sensible au nématode à kyste est utilisé comme témoin sensible. La variété Nanco est choisie à cet effet comme témoin sensible.

La lecture des boites est réalisée entre 15 et 20 jours après l'inoculation des juvéniles de deuxième stade (J2). Toutes les plantes n'ayant pas permis le développement de nématodes ou n'ayant permis que le développement de mâles sont conservées. Les plantes présentant des femelles sont éliminées.

### C. Tests de confirmation de la résistance (Etapes 2 et 3)

### Etape 2 :

Les plantes conservées à l'issue du test *in vitro* sont rempotées dans un mélange terre sable afin d'être testée une seconde fois, *in vivo,* dans un sol naturel contaminé par des kystes de *Heterodera carotae.*

Le dispositif est constitué d'un tube PVC de 20 cm de haut par 4.5 cm de diamètre contenant un sol sableux (70% de sable et 30% de terre). L'ensemble est posé sur un lit de sable de Fontainebleau, maintenu humide afin d'assurer une humidité constante Les plantes sont repiquées au contact d'un sachet de 10 kystes de *Heterodera carotae,* issus de la même population que lors du test *in vitro.* Le sachet peut être avantageusement remplacé par un apport direct de larves en solution au pied de chaque plant. Le test est réalisé sous serre, en conditions contrôlées de température 20°C ± 5 et de photopériode de 16 heures.

Deux mois après leur mise en place, les plantes sont contrôlées une à une pour leur résistance à *Heterodera carotae* par l'observation des racines, ce qui permet de déceler la présence ou l'absence de femelles, particulièrement la présence ou l'absence de kyste. Les plantes présentant des femelles développées, particulièrement des kystes, sont automatiquement éliminées puisque sensibles, les autres étant conservées puisque potentiellement résistantes.

Sur 3720 plantes différentes testées, seules 32 sont retenus à cette étape.

### Etape 3 :

Dans un troisième temps, les 32 plantes retenues sont d'abords multipliées *in vitro* selon les protocoles d'embryogenèse somatique bien connus de l'homme de l'art, voir par exemple Steward, F.C. *et al* (1958), puis quatre clones de chaque génotype sont rempotés dans un mélange terre-sable contenant des kystes de *Heterodera carotae* selon le test de l'étape 2. Seuls les génotypes ne présentant pas de nématode *Heterodera carotae* femelle, particulièrement pas de kyste, sur aucune des 4 plantes clonées sont retenues : 11 génotypes sont finalement retenus à l'issue de ces trois étapes successives. Ils sont présentés dans le tableau 1 ci-dessous.

**Tableau 1 : génotypes testés présentant une résistance à Heterodera carotae (HCR = Heterodera carotae Resistance)**

| Numéro de Génotype | Sous espèce |
|---|---|
| HCR1 | *Daucus carota* gummifer |
| HCR2 | *Daucus carota* carota |
| HCR3 | *Daucus carota* carota |
| HCR4 | *Daucus carota* carota |
| HCR5 | *Daucus carota* gummifer |
| HCR6 | *Daucus carota* carota |
| HCR7 | *Daucus carota* gummifer |
| HCR8 | *Daucus carota* commutatus |
| HCR9 | *Daucus carota* commutatus |
| HCR10 | *Daucus carota* dentatus |
| HCR11 | *Daucus carota* gummifer |

Les inventeurs ont conservé et multiplié ces 11 génotypes *in vitro.*

### Exemple 2 : Validation et identification de la résistance à Heterodera carotae

Les 11 plantes conservées à l'étape précédente sont croisées avec des plantes de carotte cultivée *Daucus carota sativus* sensibles à *Heterodera carotae* afin d'étudier l'héritabilité de la résistance.

Les plantes hybrides de première génération issues du croisement des génotypes résistants avec les plantes *Daucus carota sativus* sensibles sont semées en pots, dans de la terre prélevée dans des champs naturellement infestés par *Heterodera carotae.* Le taux moyen d'infestation d'*Heterodera carotae* de cette terre est de 16 larves par gramme de sol. Avant le semis, les graines sont décontaminées dans de l'eau à 51°C pendant 12 minutes. Les pots sont placés en serre, à une température de 20°C. Les effectifs sont de 100 graines par combinaison hybride réalisée.

Les plantes sont analysées de 70 à 90 jours après le semis, le système radiculaire de chaque plante étant soigneusement lavé puis suivi d'un jet d'eau appliqué sur les racines afin de détacher les femelles adultes et de stade J4.

On constate que 70% des plantes hybrides ayant le génotype HCR10 comme plante parente ont moins de 10 femelles au niveau des racines. Les résultats sont présentés dans la figure 1 pour quelques combinaisons hybrides ainsi que pour Nanco, la variété sensible choisie comme témoin : le tableau indique, pour chaque combinaison hybride, la distribution du nombre de femelle *Heterodera carotae* par plante, le nombre moyen de femelle *Heterodera carotae* par plante et le pourcentage de plantes présentant moins de 10 femelles *Heterodera carotae.*

Seules les plantes présentant un nombre réduit de 0 à 5 larves femelles d'*Heterodera carotae* sont retenues et autopolinisées pour produire des plantes de seconde génération.

La seconde génération est testée dans des conditions identiques à celles des tests de la première génération, semées en pots, dans de la terre prélevée dans des champs naturellement infestés par *Heterodera carotae.* Le taux moyen d'infestation d'*Heterodera carotae* de cette terre est de 55 larves par gramme de sol. Le témoin Nanco se comporte comme attendu, avec plus de 97% des plantes témoin testées présentant plus de 200 femelles *Heterodera carotae.* L'analyse des plantes de seconde génération issues du génotype HCR10 montre des niveaux de résistance élevés, plus de 80% des plantes présentant moins de 100 femelles *Heterodera carotae.* L'analyse est répétée sur deux années, les résultats étant sensiblement identiques et présentés dans la figure 2. Cette figure illustre, pour deux lignées F2 issues du génotype HCR10, la distribution du nombre de femelles, sur deux années.

De nouveau, seules les plantes présentant un nombre réduit de larves, mais cette fois de 0 à 10 larves femelle d'*Heterodera carotae* sont retenues et autopolinisées pour produire des plantes de troisième génération.

De manière similaire, les plantes de la troisième génération sont semées en pots, dans de la terre prélevée dans des champs naturellement infestés par *Heterodera carotae.* Seules les plantes présentant un nombre réduit de 0 à 10 femelles par plante de larves femelle d'*Heterodera carotae* sont retenues.

Au fil des tests et des générations, les inventeurs ont identifié le génotype HCR10 comme étant une source potentielle de résistance à *Heterodera carotae.* La multiplication du nématode est très restreinte avec ce génotype : non seulement les larves sont bloquées au stade J2 mais aussi fortement masculinisées, mettant ainsi en évidence une action nématicide desdites plantes. Les plantes de troisième génération du génotype HCR10 sont phénotypiquement encore très proches du type sauvage, elles présentent une racine blanche, très peu tubérisée, avec un feuillage cireux et luisant, un port étalé.

### Exemple 3 : Stabilité de la résistance par rapport à différentes populations Heterodera carotae

Les plantes de seconde génération issues du génotype HCR10 (F2.HCR10) sont utilisées pour cette analyse : elles sont semées en pots, dans de la terre prélevée dans des champs naturellement infestés mais d'origines géographiques multiples représentant les diverses zones de culture françaises où *Heterodera carotae* est présent. La variété sensible Nanco est utilisée comme contrôle sensible.

Les 5 populations de *Heterodera carotae* sont prélevées dans des champs de cultures infestés par *Heterodera carotae* à Créance dans la Manche, à Plouhinec dans le Morbihan, à Machecoul en Vendée, à Carpentras dans le Vaucluse et à Lambesc dans les bouches du Rhône. Leur niveau de contamination varie de 4,46 à 13,86 larves J2 par gramme de sol.

Les figures 3 et 4 décrivent le nombre moyen de femelles présentes dans les racines. Les plantes sont analysées environ 80 à 90 jours après le semis. Les plantes sont délicatement arrachées puis le système radiculaire de chaque plante est soigneusement rincé. Enfin, les kystes sont comptés sous loupe binoculaire.

Cette expérimentation montre que les plantes de seconde génération issues du génotype HCR10 présentent une résistance contre les différentes populations d'*Heterodera carotae* testées.

### Exemple 4 : Identification du mécanisme de résistance

Des clones des plantes résistantes sont repiqués dans un sol contaminé par des kystes de *Heterodera carotae.* Des clones d'un témoin sensible servent à suivre la dynamique de pénétration du nématode en contrôlant, au microscope, les racines à des temps différents après la mise en route de l'expérimentation. Les inventeurs ont constaté que les larves sont bloquées au stade J2 et également qu'elles sont fortement masculinisées.

### Exemple 5 : Essais en serre et validation

L'objectif de cette expérimentation est de tester le niveau de résistance de différentes plantes :
- plantes de seconde (F2.HCR10), de troisième (F3.HCR10) et de quatrième génération (F4.HCR10) décrites ou obtenues comme explicités dans les exemples 1 à 3 (génotypes A, B et E, respectivement),
- plantes de troisième génération provenant de croisements frère x sœur obtenues par le croisement d'une plante de seconde génération du génotype HCR10 sélectionnée pour son niveau de résistance important avec une autre plante de seconde génération du génotype HCR10 sélectionnée pour son niveau de résistance important (génotypes C, et D, correspondant à AxA),
- plantes hybrides obtenues par le croisement d'une plante de seconde génération du génotype HCR10 sélectionnée pour son niveau de résistance important avec des lignées de carotte *Daucus carota sativus* male fertiles et sensibles au nématode *Heterodera carotae* (génotypes F, G et H) ;
- le génotype I correspondant au témoin sensible Nanco.

Les plantes sont semées en trois séries distinctes (à l'exception des génotypes D et E qui ont été semés dans deux séries sur les 3), à raison d'environ 20 plantes par série pour chaque génotype, dans des pots en plastiques (8^{∗}8^{∗}8) contenant environ 380g de sol préalablement homogénéisé à la bétonnière. Le sol est naturellement infesté et provient de Créance, dans la Manche. La mesure moyenne du taux d'infestation est de 15 larves J2 par gramme de sol. L'arrosage est réalisé pot par pot, par-dessus.

La lecture du test est réalisée entre 70 et 90 jours après le semis en utilisant une méthode rapide de lecture : un comptage total des femelles (y compris les kystes) pour les plantes ayant moins de 100 femelles. Le comptage est arrêté au-delà de cette valeur de 100 femelles, et la valeur "plus de 100" est attribuée aux plantes correspondantes.

Les plantes obtenant un score inférieur à 5 femelles sont conservées et repiquées dans un mélange de terreau et de sable indemne de contamination.

Les séries sont semées à des moments distincts, entre septembre et novembre.

Les résultats sont présentés dans le tableau 2 qui regroupe, pour chaque série, les plantes en fonction de leur niveau d'infestation (« classes » de plantes présentant 0 femelles, 1 à 5 femelles, 5 à 10 femelles, etc.). Ainsi pour le génotype A, 4 plantes sur 63 plantes testées ne présentent aucune femelle, 17 plantes présentent de 1à 5 femelles etc.

Le génotype A correspond au génotype résistant (plante de seconde génération résistante issue du génotype HCR10) alors que le génotype I correspond au témoin sensible.

Pour les trois séries, le niveau du témoin est suffisamment élevé pour que le test soit considéré comme discriminant.

La moyenne M représente le nombre total de kystes comptés pour chaque plante de chaque série, divisé par le nombre de plantes analysées par génotype.

La distribution moyenne entre les différentes classes est illustrée à la figure 5, pour chacun des génotypes testés.

**Tableau 2 : nombre de kystes par plantes, pour différents génotypes.**

| | Génotypes | | | | | | | | |
|---|---|---|---|---|---|---|---|---|---|
| Classe | A | B | C | D | E | F | G | H | I |
| 0 | 4 | 5 | 4 | 13 | 5 | 0 | 1 | 0 | 0 |
| 1 à 5 | 17 | 16 | 8 | 13 | 13 | 0 | 0 | 0 | 0 |
| 5 à 10 | 11 | 14 | 4 | 6 | 6 | 1 | 0 | 2 | 0 |
| 10 à 20 | 13 | 8 | 9 | 2 | 8 | 2 | 0 | 0 | 0 |
| 20 à 30 | 8 | 0 | 5 | 3 | 4 | 3 | 2 | 1 | 1 |
| 30 à 40 | 2 | 3 | 1 | 1 | 1 | 1 | 4 | 0 | 1 |
| 40 à 50 | 1 | 3 | 2 | 0 | 2 | 7 | 3 | 1 | 0 |
| 50 à 100 | 3 | 1 | 4 | 0 | 1 | 21 | 10 | 8 | 2 |
| 100 à 1000 | 4 | 1 | 3 | 1 | 0 | 22 | 42 | 47 | 61 |
| S | 63 | 51 | 40 | 39 | 40 | 57 | 62 | 59 | 65 |
| M | 21,397 | 13,3529 | 28,275 | 12,538 | 12,075 | 85,421 | 86,371 | 92,4375 | 323,615 |

Les résultats sont les suivants, pour chaque génotype testé :
A : (F2.HCR10) génotype résistant témoin. Les résultats sont conformes à ceux obtenus dans les tests précédents, avec un niveau de résistance élevé (moyenne générale sur les 3 séries à 21.4) et la présence d'un faible pourcentage de plantes multiplicatrices (10% environ) en queue de distribution.
B: (F3.HCR10, F3 dérivée par autofécondation de la population F2.HCR10) génotype intéressant pour les 3 séries avec environ 50 % des plantes présentant moins de 10 femelles. Néanmoins, on notera la présence d'un faible pourcentage de plantes multiplicatrices (10 % environ) en queue de distribution.
E : (F4.HCR10, F4 dérivée par autofécondation de la population F3.HCR10) génotype très résistant (moyenne générale sur les 2 séries de 12.5). On note l'absence totale de plantes multiplicatrices en queue de distribution.

On constate, d'une génération à l'autre (F2, F3 puis F4) une augmentation de la résistance et la diminution du nombre de plantes multiplicatrices.
C : profil atypique avec un pourcentage de plantes presque équivalent dans toutes les classes. Ce type de profil tend à indiquer un caractère oligogénique de la résistance.
D : génotype très résistant (moyenne générale sur les deux séries de 12.5). On note l'absence presque complète de plantes multiplicatrices en queue de distribution.
F, G et H : profil identique à celui du témoin sensible I.

Ces trois génotypes (F, G, H) étant issus d'un croisement avec une lignée élite commerciale sensible à *Heterodera carotae,* les résultats montrent que la résistance est récessive.

En conséquence, le croisement entre eux d'individus issus du génotype HCR10 permettrait d'obtenir des individus homozygotes pour le caractère de résistance dans le cas d'une résistance monogénique. La résistance étant toutefois plus probablement oligogénique, les inventeurs ont poursuivi les croisements afin de stabiliser une descendance résistante.

### Exemple 6 : Tests en bac :

L'objectif de cette expérimentation est de tester le pouvoir nématicide de plantes de troisième génération du génotype HCR10.

30 plantes F3 sont plantées sur 5 lignes de semis dans des bacs (32^{∗}28^{∗}20cm) comprenant une hauteur de sol de 18cm naturellement infesté (et provenant de Créance). L'infestation est mesurée en début et en fin d'expérimentation, après l'arrachage et ce, pour chaque bac.

L'expérimentation est répétée 2 fois.

| | **Rep 1** | **Rep 1** | **Rep 2** | **Rep 2** |
|---|---|---|---|---|
| Génotype | Génotype I Sensible | Génotype B | Génotype I Sensible | Génotype B |
| Pop initiale (J2/g) | 10.92 | 18.80 | 10.6 | 22.07 |
| Pop finale (J2/g) 3 Février | 14.46 | 9.86 | - | - |
| Pop finale(J2/g) 28 Mars | | | 27.51 | 9.33 |
| Tm (Pf/Pi) | 1.32 | 0.52 | 2.59 | 0.42 |
| % | + 32.5 | - 47.53 | +159 | - 56 |

Génotype I sensible (Nanco) : conformément à ce qui était attendu, on observe une augmentation du niveau de population d'*Heterodera carotae* dans le sol, après arrachage des carottes. Cette augmentation est d'autant plus forte que le temps de présence de la culture est important (+ 32 et +159 %) ce qui correspond à la réalisation d'une génération supplémentaire dans le cas de la seconde date d'arrachage.

Génotype B : Dans les deux cas, le niveau de la population diminue d'environ 50 %. La diminution est plus marquée avec un temps de présence plus important. Les résultats pourraient être encore meilleurs si le génotype utilisé était plus homogène génétiquement : le test de l'exemple 5 montre qu'environ 10 % des plantes sont multiplicatrices pour ce génotype. Néanmoins, ces résultats sont extrêmement encourageants et sont du niveau de ceux obtenus dans le cas d'une culture piège réussie, réalisée avec une variété classique. Avec une variété classique, une destruction trop tardive entraine cependant l'effet inverse à celui recherché, c'est-à-dire un maintien ou une augmentation de la population, comme confirmé avec le génotype I sensible.

Avec d'autres génotypes (notamment les génotypes E ou D, dépourvu de plantes multiplicatrices), des valeurs proches de celles obtenues dans le cas d'une désinfection chimique (80-90 %) doivent pouvoir être obtenues, dans ce test.

### Exemple 7 : Validation du pouvoir nématicide :

Un test artificiel en serre a été mis en place afin de mesurer le niveau du pouvoir nématicide d'une culture de carotte résistante à *Heterodera carotae,* identifiée dans les précédents exemples.

### Protocole :

Ce test est réalisé en jardinières contenant du sol naturellement contaminé prélevé dans la région de Créance (Normandie). Le critère mesuré est le taux d'infestation du sol (nombre de kystes) au moment du semis puis à différentes dates après semis, ce qui permet de mesurer un taux de multiplication du nématode *Heterodera carotae* au cours de la culture.

Le dispositif utilisé est de type randomisation en blocs complets avec 2 répétitions par génotype.
Matériel Végétal testé :
   Témoin sensible : Nanco
Plantes résistantes :
   - 2 lots différents de F3.HCR10 (génotype B dans l'exemple 5): F3.HCR10a et F3.HCR10b ;
   - 2 lots différents de plantes issues de F3.HCR10 par multiplication : F3.HCR10.Ma et F3.HCR10.Mb

### Résultats :

Les inventeurs ont observé une très mauvaise germination lors de la réalisation de ce test : 33-34% pour Nanco et 0 à 14% pour les 4 lignées F3 résistantes, dont 0% pour la lignée F3.HCR10b. Ce mauvais taux de germination, observé pour toutes les plantes testées, est probablement lié à de mauvaises conditions lors de la germination.

Les inventeurs ont observé qu'avec Nanco, le témoin sensible, l'augmentation de la population de nématodes est de 430% après 6 mois de culture, et ce malgré les mauvaises conditions de germination.

Pour les lignées F3.HCR10a et F3.HCR10.Ma, il est observé une diminution du taux d'infestation du sol d'environ 20% (F3.HCR10.Ma) à environ 40% (F3.HCR10a), après 6 mois de culture, et malgré les mauvaises conditions de germination.

### Conclusion :

Le pouvoir nématicide est bien confirmé par ce test.

Cependant, étant donné la très mauvaise germination lors de ce test, il n'est pas possible de conclure de manière précise sur le niveau du pouvoir nématicide de ce matériel résistant à *Heterodera carotae.*

### Exemple 8 : Création de lots de graines des génotypes les plus résistants.

Une multiplication des meilleures F3 identifiées a été réalisée sous grandes cages et sous tunnel plastique (la fécondation se faisant de manière aléatoire par les insectes), en vue du dépôt de graines, pour obtenir :
- une population synthétique créée à partir de l'intercroisement des plantes F3 les plus résistantes ;
- une population F4.HCR10.M, correspondant à une multiplication de la lignée F4.HCR10 décrite à l'exemple 5 dont le pouvoir nématicide a déjà été confirmé.

Les graines déposées le 20 janvier 2015 au NCIMB, sous le numéro d'accession NCIMB 42351 et la référence Daucus carota DCHR1, correspondent à une multiplication de la population F4.HCR10.M, baptisée F4.HCR10.MM.

### Exemple 9 : Nouvelle validation du pouvoir nématicide en jardinière

Ce test est réalisé afin de tester l'effet nématicide d'une population de carotte sur *Heterodera carotae.* Il est réalisé dans des jardinières, chaque génotype étant testé dans une jardinière sur deux rangées. Afin d'assurer une robustesse du test, deux répétitions sont réalisées.

Le suivi de l'effet nématicide est opéré à l'aide du comptage du nombre de kystes dans la terre infectée à l'aide de prélèvements avant semis, à la mi-test 4 mois après semis, et à la fin du test 6 mois après semis, ce qui permet de mesurer un taux de multiplication du nématode *Heterodera carotae* au cours de la culture.

Inoculum : Inoculum naturel : Terre provenant d'un champ infesté de nématodes *Heterodera carotae.* Cet inoculum est bien homogénéisé en bétonnière.

Préparation des jardinières : des jardinières trouées de 29 litres H : 20cm, L : 98cm, I : 25cm (1 jardinière par génotype et par répétition) sont remplies de strictement la même quantité de terre.

### Matériel végétal testé :

Lot 1. Nanco : témoin sensible
Lot 2. lignée F4 résistante (F4.HCR10.M, voir exemple 8)
Lot 3. une population synthétique, telle que décrite à l'exemple 8
Lot 4. lignée Pseudo F3 résistante
Lot 5. lignée pseudo F3 résistante

Semis : les graines sont semées en laissant un espace de 5 cm entre les graines et le bord, afin d'éviter un effet de bordure. Le semis se réalise sur deux rangées parallèles de 70 cm avec 60 graines par rangées réparties de manière homogène sur toute la longueur. Prélèvements : Durant la durée du test, 3 prélèvements de 3 échantillons sur chaque jardinière sont réalisés. Le prélèvement n°1 correspond au prélèvement avant le semis, le n°2 correspond au prélèvement 4 mois après semis et le n°3 correspond au prélèvement 6 mois après semis.

### Résultats :

- Les inventeurs ont observé une bonne germination pour l'ensemble des lots testés, excepté le lot 4 (25%), mais un très mauvais développement des plantes du témoin sensible Nanco (retard très important de croissance). Du fait du retard de croissance du témoin sensible, les résultats de multiplication des nématodes ne sont pas conformes aux résultats obtenus les années précédentes dans ce genre de test. En effet, le retard de croissance conduit à l'introduction d'un biais dans les résultats : alors que dans tous les tests précédents il a été observé une multiplication du nématode dans le sol (de 159 à 430% pour les témoins sensibles), on observe ici une diminution des nématodes de 52%. Le mauvais développement des racines a probablement eu un impact sur le cycle du nématode.
- 3 des génotypes testés permettent une diminution du taux d'infestation du sol de 62% à 87% après 6 mois de culture ;
- pour l'un des génotypes (Lot 5, pseudo F3 résistante), il y a une augmentation de la population de nématode de 60% après 6 mois de culture, ce qui est inattendu.

Les résultats sont illustrés à la figure 6. Les tableaux ci-après rapportent l'analyse de la variance et les résultats du Test de Newman-Keuls au seuil 5%.

### Analyse de variance :

| | S.C.E | DDL | C.M. | TEST F | PROBA |
|---|---|---|---|---|---|
| VarTOTALE | 28367.288735 | 9 | 3151.920971 | | |
| Var.FACTEUR 1 | 26750.325501 | 4 | 6687.581375 | 25.584440 | 0.006094 |
| Var.BLOCS | 571.393143 | 1 | 571.393143 | 2.185958 | 0.212694 |
| VAR.RESIDUELLE 1 | 1045.570091 | 4 | 261.392523 | | |

### Test de Newman-Keuls au seuil 5%

| Modalité | Moyenne | Groupes homogènes |
|---|---|---|
| Lot 5 | 59.842520 | A |
| Lot 1:NANCO | - 51.968504 | B |
| Lot 4 | - 62.204724 | B |
| Lot 3 | - 64.566929 | B |
| Lot 2 | - 86.614173 | B |

### Conclusions :

Le pouvoir nématicide lié à cette résistance est à nouveau confirmé par le test réalisé.

Le génotype F4.HCR10.M résistant à *Heterodera carotae,* avec un pouvoir nématicide de l'ordre de 80% après 4 à 6 mois de culture, permet d'obtenir un effet comparable à un traitement chimique (ayant une efficacité de 80-90%).

Ce génotype avait déjà montré le meilleur pouvoir nématicide dans le test précédent ; des graines obtenues par multiplication de ce génotype ont été déposées au NCIMB sous le numéro NCIMB 42351.

### BIBLIOGRAPHIE

Murashige T and Skoog F (1962) A revised medium for rapid growth and bioassays with tobacco tissue cultures. Physiol Plant 15(3): 473-497.
Steward, F.C., Mapes, M.O., and Smith, J. (1958). Growth and organized development of cultured cells. I, Il & III. Growth and division of freely suspended cells. Am. J. Bot. 45

## Revendications

1. Utilisation de plantes *Daucus carota* qui n'appartiennent pas à la sous-espèce des carottes cultivées *Daucus carota sp.* Sativus, pour désinfecter un milieu de culture infesté par des nématodes *Heterodera carotae,* lesdites plantes présentant un effet nématicide.

2. Utilisation selon la revendication 1, où l'effet nématicide est une réduction du nombre de larves juvéniles J2 et/ou une réduction du nombre de kystes et/ou une masculinisation supérieure à 50 % des larves du nématode *Heterodera carotae* lors d'une culture d'au moins 4 mois desdites plantes.

3. Utilisation selon la revendication 1, où lesdites plantes sont issues des graines déposées au NCIMB sous le numéro d'accession NCIMB 42351.

4. Utilisation selon l'une quelconque des revendications 1 à 3, où le milieu de culture infesté est de la terre ou du sable.

5. Utilisation selon l'une quelconque des revendications 1 à 4, permettant de réduire la population de nématodes *Heterodera carotae* au sein du milieu de culture d'au moins 40 %.

6. Utilisation selon la revendication 5, permettant de réduire la population de nématodes *Heterodera carotae* au sein du milieu de culture d'au moins 50%.

7. Utilisation selon la revendication 5, permettant de réduire la population de nématodes *Heterodera carotae* au sein du milieu de culture d'au moins 60%.

8. Utilisation selon l'une quelconque des revendications 1 à 4, permettant de réduire d'au moins 50% le nombre de kystes ou de nématodes femelles du milieu de culture.

9. Utilisation selon l'une quelconque des revendications 1 à 8, permettant de réduire le niveau d'infestation du milieu de culture à moins d'une larve J2 femelle d'*Heterodera carotae* par gramme de milieu.

10. Méthode de réduction de la population de nématodes *Heterodera carotae* dans un milieu de culture infesté, comprenant la culture de plantes *Daucus carota* qui n'appartiennent pas à la sous-espèce des carottes cultivées *Daucus carota sp. Sativus,* pendant une durée d'au moins 4 mois, où lesdites plantes présentent un effet nématicide sur lesdits nématodes.

11. Méthode selon la revendication 10, où ladite culture est d'une durée d'au moins 5 mois.

12. Méthode selon la revendication 11, où ladite culture est d'une durée d'au moins 6 mois.

13. Méthode selon l'une quelconque des revendications 10 à 12, où ladite culture est réalisée à une densité de 0,8 à 4 millions de graines par hectare.

## Patentansprüche

1. Verwendung von *Daucus* carota-Pflanzen, die nicht zur Unterart der Kulturkarotten *Daucus carota sp.* Sativus gehören, zur Desinfektion eines von *Heterodera carotae*-Nematoden befallenen Kulturmediums, wobei diese Pflanzen eine nematizide Wirkung haben.

2. Verwendung nach Anspruch 1, wobei die nematizide Wirkung in einer Verringerung der Anzahl der juvenilen Larven J2 und/oder einer Verringerung der Anzahl der Zysten und/oder einer Maskulinisierung von mehr als 50 % der Larven der *Heterodera carotae-*Nematoden während einer Kultur von mindestens 4 Monaten dieser Pflanzen besteht.

3. Verwendung nach Anspruch 1, wobei die Pflanzen von Samen stammen, die bei NCIMB unter der Zugangsnummer 42351 der NCIMB hinterlegt sind.

4. Verwendung nach einem der Ansprüche 1 bis 3, wobei es sich bei dem befallenen Kulturmedium um Erde oder Sand handelt.

5. Verwendung nach einem der Ansprüche 1 bis 4, die es ermöglicht, die Population von *Heterodera carotae*-Nematoden im Kulturmedium um mindestens 40 % zu reduzieren.

6. Verwendung nach Anspruch 5, die es ermöglicht, die Population von *Heterodera carotae-*Nematoden im Kulturmedium um mindestens 50 % zu reduzieren.

7. Verwendung nach Anspruch 5, die es ermöglicht, die Population von *Heterodera carotae-*Nematoden im Kulturmedium um mindestens 60 % zu reduzieren.

8. Verwendung nach einem der Ansprüche 1 bis 4, die es ermöglicht, die Anzahl der Zysten oder der weiblichen Nematoden im Kulturmedium um mindestens 50 % zu reduzieren.

9. Verwendung nach einem der Ansprüche 1 bis 8, die es ermöglicht, die Befallsrate des Kulturmediums auf weniger als eine weibliche Larve J2 von *Heterodera carotae* pro Gramm Medium zu reduzieren.

10. Verfahren zur Reduzierung der Population von *Heterodera carotae*-Nematoden in einem befallenen Kulturmedium, umfassend die Kultur von *Daucus* carota-Pflanzen, die nicht zur Unterart der Kulturkarotten *Daucus carota sp. Sativus* gehören, für einen Zeitraum von mindestens 4 Monaten, wobei diese Pflanzen eine nematizide Wirkung auf die Nematoden haben.

11. Verfahren nach Anspruch 10, wobei die Kultur mindestens 5 Monate dauert.

12. Verfahren nach Anspruch 11, wobei die Kultur mindestens 6 Monate dauert.

13. Verfahren nach einem der Ansprüche 10 bis 12, wobei die Kultur mit einer Dichte von 0,8 bis 4 Millionen Samen pro Hektar durchgeführt wird.

## Claims

1. Use of *Daucus carota* plants which do not belong to the sub-species of cultivated carrots *Daucus carota* sp. *sativus,* in order to disinfect a cultivation medium infested with *Heterodera carotae* nematodes, said plants exhibiting a nematicidal effect.

2. Use according to claim 1, wherein the nematicidal effect is a reduction in the number of J2 juvenile larvae and/or a reduction in the number of cysts and/or a masculinization of more than 50% of *Heterodera carotae* nematode larvae during cultivation of said plants for at least 4 months.

3. Use according to claim 1, wherein said plants are obtained from seeds deposited with the NCIMB under accession number NCIMB 42351.

4. Use according to any one of claims 1 to 3, wherein the infested cultivation medium is earth or sand.

5. Use according to in any one of claims 1 to 4, enabling the population of *Heterodera carotae* nematodes in the cultivation medium to be reduced by at least 40%.

6. Use according to claim 5, enabling the population of *Heterodera carotae* nematodes in the cultivation medium to be reduced by at least 50%.

7. Use according to claim 5, enabling the population of *Heterodera carotae* nematodes in the cultivation medium to be reduced by at least 60%.

8. Use according to any one of claims 1 to 4, enabling the number of cysts or female nematodes in the cultivation medium to be reduced by at least 50%.

9. Use according to any one of claims 1 to 8, enabling the level of infestation of the cultivation medium to be reduced to less than one J2 female *Heterodera carotae* larva per gram of medium.

10. A method for reducing the population of *Heterodera carotae* nematodes in an infested cultivation medium, comprising the cultivation of *Daucus carota* plants which do not belong to the sub-species of cultivated *Daucus carota* subsp. *sativus* carrots, during at least 4 months, wherein said plants have a nematicidal effect on said nematodes.

11. The method according to claim 10, wherein said cultivation is of a duration of at least 5 months.

12. The method according to claim 11, wherein said cultivation is of a duration of at least 6 months.

13. The method according to any one of claims 10 to 12, wherein said cultivation is carried out at a density of 0.8 to 1.4 million seeds per hectare.
